Europäisches Patentamt

European Patent Office

Office européen des brevets

(11) Publication number: **0 093 101**
**A2**

# EUROPEAN PATENT APPLICATION

(21) Application number: 83850105.4

(22) Date of filing: 22.04.83

(51) Int. Cl.³: **A 61 M 25/00**

(30) Priority: 22.04.82 PC T/SE82/00127
07.06.82 SE 8203499

(43) Date of publication of application:
02.11.83 Bulletin 83/44

(84) Designated Contracting States:
IT

(71) Applicant: Gustavsson, Bengt
Bergsbogatan 29
S-421 79 Västra Frölunda(SE)

(71) Applicant: Curelaru, Ioan
Dr. Lindsgatan 3
S-413 25 Göteborg(SE)

(71) Applicant: Linder, Lars-Erik
Valebergsvägen 319
S-427 00 Billdal(SE)

(72) Inventor: Gustavsson, Bengt
Bergsbogatan 29
S-421 79 Västra Frölunda(SE)

(72) Inventor: Curelaru, Ioan
Dr. Lindsgatan 3
S-413 25 Göteborg(SE)

(72) Inventor: Linder, Lars-Erik
Valebergsvägen 319
S-427 00 Billdal(SE)

(74) Representative: Roth, Ernst Adolf Michael et al.
GÖTEBORGS PATENTBYRA AB Box 5005
S-402 21 Göteborg(SE)

(54) A device for introducing a catheter into a blood vessel.

(57) An introducer-cannula for introducing a catheter into a blood-vessel. The introducer-cannula (10) is arranged on the outside of a needle (13) and is introduced into the blood-vessel together with this. The introducer-cannula has a front end with a reduced or tapering diameter, said front end connects tightly to the needle. The front end is provided with an expandable, e.g. slotted portion (16), which can be severed by the tip to a catheter (18) having a diameter exceeding that of said front end and permit the passage of the catheter through the introducer-cannula (10) into the blood-vessel after removal of the needle (13). In connection to said expandable portion (16) there is preferably arranged a line of weakness (17) extending over the rest of the length of the introducer-cannula and along which the introducer-cannula can be severed for facilitating its withdrawal from the catheter (18) when this has been located in the blood-vessel.

FIG 2

**0093101**

<u>A device for introducing a catheter into a blood-vessel</u>

<u>Background of the invention</u>

The present invention refers to a device for introducing a catheter into a blood-vessel and comprising a needle for puncturing the blood-vessel and an introducer-cannula intended to be introduced into the blood-vessel together with the needle and through which the catheter-cannula is intended to be introduced into the blood-vessel after removal of the needle, said introducer-cannula having a front end with a reduced or tapering outer diameter and which tightly connects to the needle.

Plastic catheters are inserted into practically all body cavities and are used for treatment and examination purposes within the modern medical service. In order to introduce especially soft catheters in for example a blood-vessel special devices are needed and there are suggested a number of methods, which however all suffer from certain drawbacks.

Among these method it can be mentioned "the catheter-over-needle" technique, i.e. the catheter is passed on the outside of the needle and this method is used for central venous catheters. This method however involves a great risk for injuries on the catheterized and neighbouring organs owing to the long needle (16-18 cm.), which besides make the device difficult to handle.

According to another method the catheter is passed through the needle, "catheter-through-steel-needle" technique, at which the needle either may remain or be removed after the introduction of the catheter. Also this methods involves a great risk for injuries and a risk for catheter embolism, by the fact that the catheter easily is damaged by the sharp needle tip.

It is also known to use an outer introducer-cannula by Teflon(R), which is introduced into the blood-vessel together with the needle. After the puncture of the blood-vessel the needle is removed and the catheter is then introduced into the blood-vessel through the introducer-cannula. This method however, easily causes injuries at the introduction owing to the considerable diameter of the introducer-cannula.

According to a further method, called Seldinger's technique, a stiff wire is used which is introduced into the blood-vessel through the needle, after which the catheter is introduced over the wire, which act as a guide for the catheter. When the catheter has been introduced the wire is withdrawn . It is however a complicated method, which often makes it necessary to perform a skin incision and to dilate the path for permitting entrance of the catheter.

According to a developement of this technique, called Desilet's and Hoffmanns technique, there is provided a dilatation of the puncture hole by means of a special introducer-cannula, through which the catheter then is introduced. It is however also a complicated method.

Through SE-B 414.453 there is previously known an introducer-cannula for introducing catheters into a blood-vessel and which is provided with a chamfered front end which tightly connects to the needle in order to facilitate the introduction of the introducer-cannula into the blood-vessel. The catheter is introduced into the blood-vessel through the introducer-cannula after the removal of the needle, after which the introducer-cannula is removed from the blood-vessel by displacing it backwards along the catheter. The diameter of the catheter is limited by the inside diameter of the introducer-cannula, which is substantially constant except at a rear enlarged section. The introduction of the catheter, which has substantially the same outer diameter as the inside diameter of the introducer- cannula will be difficult because of the friction against the inner walls of the introducer-cannula along the substantial length thereof. Besides the removal of the introducer-cannula from the blood-vessel can cause problems if the outer diameter of the catheter is not considerably smaller than the inside diameter of the introducer-cannula.

Through DE-A 29.36.655 it is previously known a device for introducing a sampling instrument, urinary catheter or the like into a body cavity, e.g. the urethra, without the risk for contamination by germs. The device comprises a sleeve, the front end of which is closed and can be broken by means of the instrument, the catheter or the like. Such an introducer sleeve could not be used in connection with the introduction of venous catheters into a blood-vessel, where you firstly have to penetrate the skin. Besides it will not be possible to break the closed end of the sleeve by a soft venous catheter.

### The purpose and most important features of the invention

The purpose of the present invention is to provide an introducer-cannula adapted for the introduction of short catheters into peripheral blood-vessels as well as long central venous catheters and which shall fulfil the following demands:

a) it shall be easily inserted into a blood-vessel and not injure this or the neighbouring organs,
b) it shall be easily removable from the blood-vessel in order to prevent ackumulation of blood and growth of germs between the introducer-cannula and the catheter,
c) it shall not damage the catheter and by that cause catheter embolism,
d) it shall permit the insertion of both soft and stiff catheters,
e) it shall not require special tools or operations as stiff wires, skin incisions or dilitation of the puncture hole,
f) it shall be cheap to manufacture.

These demands have according to the invention been fulfilled by the fact that the inside diameter of said front end is likewise reduced or tapering and that the front end is provided with an expandable portion intended to be severed by the catheter at the introduction thereof and to permit the passage of a catheter having an outer diameter exceeding the inside diameter of the front end of the introducer-cannula.

Fig. 1 is a longitudinal section through an introducer-cannula according to the invention,

Fig. 2 is a longitudinal section of the whole device including the needle,

Fig 3 is a longitudinal section according to fig. 2 of another embodiment,

Fig. 4a-c show schematically in a view from above different steps of the introduction of a catheter through an introducer-cannula according to the invention,

Fig. 5-8 show in perspective the front end of different embodiments of the introducer-cannula according to the invention.

### Description of embodiments

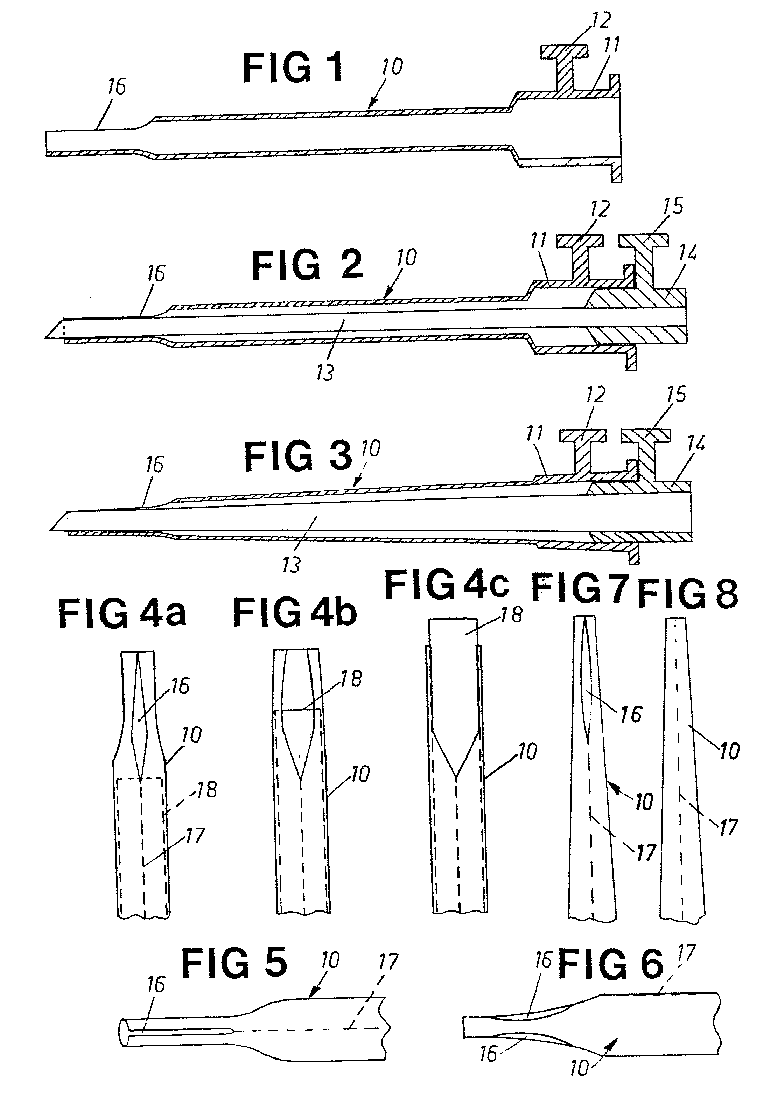

The introducer-cannula 10 is manufactured by a stiff plastic material, for example Teflon(R) and has a reduced front end. Alternatively the whole introducer-cannula 10 tapers towards the front end as is shown in fig. 3,6 and 7. The opposite end of the introducer-cannula 10 is shaped as a holder member 11 and is provided with a grip 12 for facilitating the insertion. A needle 13 is intended to be received within the introducer-cannula as is shown i fig. 2. The reduced front end of the introducer-cannula 10 connects tightly to the needle 13 for facilitating the insertion. Also the needle is provided with a holder member 14 with a grip 15. It would also be possible, as is shown in fig. 3, to give the needle 13 a shape corresponding to the introducer-cannula 10, i.e. a reduced front end or alternatively tapering over its entire length.

The front tip of the introducer-cannula is provided with a slit 16, which either as is shown in fig. 4 extends all the way to said end or as is shown in fig. 1-4 and 6-7 respectively ends shortly before the end. The embodiment shown in fig. 6 is provided with two slits 16 on opposite sides of the introducer-cannula 10. A line of weakness 17 connects to the slit 16, for example a groove, perforation or the like, which extends along the entire introducer-cannula 10 including its holder member 11 and along which the introducer-cannula can be splitted at its withdrawal. In the embodiment according to fig. 8 the introducer-cannula 10 has no slit and is instead provided with a line of weakness 17 extending over its entire length.

The device is used in the following way. The blood-vessel, either a peripheral or central vein or artery, is punctured by means of the tip of the needle 13 and the whole device comprising needle 13 and introducer-cannula 10 is inserted together into the blood-vessel to the desired position. After that the needle 13 is withdrawn. The catheter 18 is then introduced through the introducer-cannula 10, at which the tip of the catheter forces the front end of the introducer-cannula 10 apart, as is illustrated in fig. 4a-c, and the catheter can pass through the introducer-cannula and into the blood-vessel. The introducer-cannula is easily removed from the catheter by severing it along the line of weakness 17.

The device according to the invention has a number of important advantages:

a) it permits a careful insertion into the blood-vessel with less risk for injuries as compared to most of the previously known methods,
b) it can easily be removed from the blood-vessel,
c) no risk for damages on the catheter and catheter embolism since the catheter is inserted through a non-sharp Teflon(R)-sleeve,
d) it permits the introduction of both soft and stiff catheters, both periperal and central venous catheter and both artery and venous catheters,

e) it permits a simple introduction, the introducer-cannula dilates the blood-vessel at the same time and no guide wires, scalpels or the like are needed,

f) it is cheap to manufacture.

The invention is of course not limited to the above described and shown embodiments, but can be modified within the scope of the claims.

P13397EP-6019/B1-830510

## CLAIMS

1. A device for introducing a catheter into a blood-vessel and comprising a needle (13) for puncturing the blood-vessel and an introducer-cannula (10) intended to be introduced into the blood-vessel together with the needle and through which the catheter (18) is intended to be introduced into the blood-vessel after removal of the needle, said introducer-cannula (10) having a front end with a reduced or tapering outer diameter and which tightly connects to the needle (1),
c h a r a c t e r i z e d   i n,
that the inside diameter of said front end is likewise reduced or tapering and that the front end is provided with an expandable portion (16) intended to be severed by the catheter (18) at the introduction thereof and to permit the passage of a catheter having an outer diameter exceeding the inside diameter of the front end of the introducer-cannula (10).

2. A device according to claim 1,
c h a r a c t e r i z e d   i n,
that said expandable portion comprises a slit (16).

3. A device according to claim 1 or 2,
c h a r a c t e r i z e d   i n,
that in connection to said expandable portion (16) there is arranged a line of weakness (17) extending over the rest of the length of the introducer-cannula (10).

4. A device according to any of the preceding claims,
c h a r a c t e r i z e d   i n,
that the needle (13) is provided with a front end having a reduced or tapering diameter corresponding to that of the introducer-cannula (10).

0093101

FIG 1

FIG 2

FIG 3

FIG 4a   FIG 4b   FIG 4c   FIG 7   FIG 8

FIG 5   FIG 6